# EUROPÄISCHE PATENTSCHRIFT

(11) **EP 2 892 481 B1**
(45) Veröffentlichungstag und Bekanntmachung des Hinweises auf die Patenterteilung: **08.08.2018**
(21) Anmeldenummer: 13753897.1
(22) Anmeldetag: 03.09.2013
(51) Int. Cl.: A61F 13/02, A61L 31/14

(54) **WUNDPFLASTER, RESORBIERBARE WUNDAUFLAGE UND SET FÜR DIE HERSTELLUNG WENIGSTENS EINES WUNDPFLASTERS**
ADHESIVE PLASTER, RESORBABLE WOUND DRESSING AND SET FOR PRODUCING AT LEAST ONE ADHESIVE BANDAGE
PANSEMENT POUR PLAIES, ÉLÉMENT RÉSORBABLE DE CONTACT AVEC LA PLAIE, ET ENSEMBLE PERMETTANT DE FABRIQUER AU MOINS UN PANSEMENT POUR PLAIES

(30) Priorität: 07.09.2012 DE 102012215931
(43) Veröffentlichungstag der Anmeldung: 15.07.2015
(73) Patentinhaber: PolyMedics Innovations GmbH, 73770 Denkendorf (DE)
(72) Erfinder: PLANCK, Constanze, 72622 Nürtingen (DE); MÜLLER, Erhard, 70565 Stuttgart (DE); HIERLEMANN, Helmut, 73033 Göppingen (DE)
(74) Vertreter: Prinz & Partner mbB
(86) Internationale Anmeldenummer: PCT/EP2013/068206
(87) Internationale Veröffentlichungsnummer: WO 2014/037355

(56) Entgegenhaltungen:
- DE-A1-102004 063 599
- US-A1- 2008 167 729

## Beschreibung

Die Erfindung betrifft ein Wundpflaster mit einer Wundauflage, die im Applikationsfall mittels einer Wirkfläche mit einer Hautwunde in Kontakt gelangt, sowie mit einem flächigen Trägermaterial, das die Wundauflage auf einer zur Wirkfläche der Wundauflage entgegen gerichteten Rückfläche zumindest abschnittsweise überdeckt, und das die Wundauflage mit wenigstens zwei zumindest weitgehend gegenüberliegenden Haftflächen überragt, die zur Fixierung des Trägermaterials auf menschlicher oder tierischer Haut vorgesehen sind. Die Erfindung betrifft auch ein Set für die Herstellung wenigstens eines Wundpflasters zur Behandlung von Hautwunden mit wenigstens einem Trägermaterialzuschnitt und wenigstens einem größenmäßig auf den wenigstens einen Trägermaterialzuschnitt abgestimmten Wundauflagezuschnitt aus resorbierbarem Material.

Wundpflaster zur Behandlung von Hautwunden sind allgemein bekannt. Sie werden sowohl für den Hausgebrauch als auch in Arztpraxen oder Kliniken verwendet und dienen insbesondere zur Behandlung von Schürfwunden und Injektionseinstichstellen. Ein derartiges Wundpflaster weist ein flächiges Trägermaterial auf, an dessen Unterseite eine nicht resorbierbare Wundauflage fixiert ist. Seitlich der Wundauflage weist die Unterseite des Trägermaterials Haftflächen in Form von Klebeflächen auf, die zur Fixierung des Wundpflasters auf der Haut dienen. Dabei wird das Wundpflaster mit seiner Wundauflage über die offene Wunde gelegt und anschließend mittels des Trägermaterials auf die um die Wunde befindliche gesunde Haut aufgeklebt. Die Wundauflage ist meist derart konzipiert, dass kein Verkleben der Wundauflage mit der Wunde möglich ist, um die Wundheilung durch den notwendigen häufigen Verbandswechsel nicht zu stören.

Ein Formkörper zur medizinischen Behandlung von Wunden ist aus der EP 1919528 B1 bekannt. Der bekannte Formkörper kann als resorbierbare Wundauflage gestaltet sein. Diese Wundauflage ermöglicht die Abführung von Wundflüssigkeit aus Wunden und ist damit insbesondere für die leistungsstarke Behandlung und Versorgung von großflächigen und tiefen Wunden geeignet. Außerdem unterstützt ein Wundverband aufgrund seiner Zusammensetzung die Wundheilung.

Aufgabe der Erfindung ist es, ein Wundpflaster und ein Set der eingangs genannten Art zu schaffen, das mit geringem Aufwand eine einfache Behandlung und eine schnelle Wundheilung von insbesondere klein- und mittelflächigen Hautwunden ermöglicht.

Diese Aufgabe wird für das Wundpflaster dadurch gelöst, dass die Wundauflage aus resorbierbarem Material besteht und mittels einer Haltestruktur lösbar mit dem Trägermaterial verbunden ist und die Haltestruktur lösbare Verbindungsstellen zwischen Trägermaterial und Wundauflage aufweist, die die Wundauflage in nicht appliziertem Zustand am Trägermaterial sichern. Die resorbierbare Wundauflage verbleibt solange auf der Wunde, bis die Wundheilung abgeschlossen ist. Die Haltestruktur kann erfindungsgemäß am Trägermaterial oder an der Wundauflage vorgesehen sein. Es ist auch möglich, die Haltestruktur durch Strukturbereiche zu bilden, die komplementär zueinander sowohl an der Wundauflage als auch an dem Trägermaterial vorgesehen sind und miteinander in Wirkverbindung treten. Als Wundkontaktmaterial, das die resorbierbare Wundauflage bildet, werden vorzugsweise resorbierbare Materialien eingesetzt, wie sie aus der EP 1181941 A2 oder der EP 1919528 B1 bekannt sind. Zur näheren Erläuterung dieser Materialien wird auf die Offenbarung in den genannten Druckschriften EP 1181941 A2 und EP 1919528 B1 verwiesen. Die erfindungsgemäße Idee ist es, durch die Verbindung der resorbierbaren Wundauflage mit dem Trägermaterial eine schnelle und einfache Aufbringung der Wundauflage auf die Hautwunde zu ermöglichen. Die Lösbarkeit der Verbindung zwischen Wundauflage und Trägermaterial durch die Haltestruktur gewährleistet ein einfaches Ablösen des Trägermaterials von der Wundauflage, die auf der Wunde verbleibt. So ist es insbesondere möglich, bereits nach einem relativ kurzen Zeitraum von ein bis zwei Tagen das Trägermaterial zu entfernen, wohingegen die mit der Wunde zumindest teilweise verklebte Wundauflage auf der Wunde verbleibt. Eine Irritation der Wundheilung, die bei bekannten Wundpflastern durch ein Entfernen der mit dem Trägermaterial fest verbundenen Wundauflage entstehen konnte, wird hierdurch vermieden. Durch die mögliche Entfernung des Trägermaterials und den gleichzeitigen Verbleib der resorbierbaren Wundauflage auf der Wunde erfolgt zudem eine schnellere Heilung der Wunde, da nach Entfernung des Trägermaterials eine verbesserte Wechselwirkung zwischen Umgebungsluft und Wundauflage ermöglicht ist. Die Wundauflage ist aus resorbierbarem, körperverträglichen Kunst- oder Naturmaterial als Folie, als textiles Flächengebilde in Form eines Gewebes, eines Gestricks, eines Gewirkes oder eines Vliesstoffes oder Kombinationen daraus oder als offenporiger Formkörper, insbesondere in Form einer Membran, hergestellt. Vorzugsweise weist das Wundauflagematerial eine Wasserdampfdurchlässigkeit auf, die einer Wasserdampfdurchlässigkeit der menschlichen Haut entspricht. Die erfindungsgemäße Lösung eignet sich zur Versorgung sowohl von menschlichen als auch von tierischen Hautwunden kleiner und mittlerer Größe. Dadurch, dass die resorbierbare Wundauflage nach Applikation des Wundpflasters mit der Wunde verklebt, und auch nach Entfernung des Trägermaterials in diesem Zustand verbleibt, ist eine ungestörte Regeneration des Hautdefektes ermöglicht. Nach Abschluss der Wundheilung löst die Wundauflage sich selbständig ab oder kann schmerzfrei abgezogen werden. Der Verbleib der Wundauflage auf der Wunde gewährleistet sowohl einen bakteriellen als auch einen mechanischen Schutz der Hautwunde. Die Entfernung des Trägermaterials, das eine gegenüber dem Wundauflagematerial gleiche oder reduzierte Wasserdampfdurchlässigkeit aufweisen kann, ermöglicht eine verbesserte Abführung von Wundflüssigkeit an die Umgebung, wodurch die Wundheilung schneller erfolgt im Vergleich zu einem auf der Wunde vollständig verbleibenden Wundpflaster einschließlich Trägermaterial. Die erfindungsgemäße Lösung eignet sich insbesondere zur Versorgung von kleinbis mittelflächigen Hautläsionen aller Art, insbesondere Schürfwunden, OP-Wunden, Injektionseinstichstellen, Drainageeinstichstellen, klein- und mittelflächigen Hautverbrennungen, Ekzemen und von Allergien. Die resorbierbare Wundauflage weist vorzugsweise eine antibakterielle Wirkung auf und ist atmungsaktiv. Das Wundauflagematerial ist zur guten Anpassungsfähigkeit an die Haut plastisch und/oder elastisch verformbar, wobei vorzugsweise die Verformbarkeit an das Verformungs- und Dehnungsvermögen der Haut angepasst ist. Entsprechende Eigenschaften des Wundauflagematerials können durch Polymereigenschaften eingestellt bzw. angepasst werden. Alternativ oder ergänzend kann die Struktur des Wundauflagematerials, insbesondere bei Gestaltung aus einem textilen Flächengebilde, vorzugsweise einem Vliesstoff, die gewünschten Eigenschaften bewirken. Das Trägermaterial ist vorzugsweise elastisch verformbar. Das Trägermaterial kann durch ein textiles Flächengebilde in Form einer Web- oder Maschenware oder eines Vliesstoffes oder in Form einer Kunststofffolie, insbesondere aus PVC oder PUR, jeweils ein- oder mehrschichtig gestaltet sein. Erfindungsgemäße Wundpflaster weisen vorzugsweise Wundauflagen mit Wirkflächen im Bereich zwischen 10 mm² und 1000 cm² auf. Das flächige Trägermaterial kann die Wundauflage allseitig oder lediglich an zwei gegenüberliegenden Seiten überragen. An den überragenden Bereichen sind an der zur Haut gewandten Unterseite des Trägermaterials entsprechende Haftflächen vorgesehen. Erfindungsgemäße Wundpflaster können als Zuschnitte oder auch als endlose, zu einer Rolle aufgewickelte Bänder gestaltet sein, von denen entsprechende Streifen als Wundpflasterzuschnitte abgetrennt werden. Bei derartigen Wundpflasterrollen erstrecken sich die Wundauflagen durchgängig über die Länge des Trägermaterials.

In Ausgestaltung der Erfindung umfasst die Haltestruktur wenigstens in einem Überdeckungsbereich zwischen dem Trägermaterial und der Wundauflage eine hydrophobe Struktur, die im Applikationsfall eine Aufnahme von Wundflüssigkeit durch das Trägermaterial verhindert. Dadurch ist gewährleistet, dass das Trägermaterial nach Aufbringen des Wundpflasters auf die Hautwunde nicht mit der resorbierbaren Wundauflage verklebt. Das Trägermaterial hat kein Aufsaugvermögen von Wundflüssigkeit. Ein Anhaften des Trägermaterials an der Wundauflage und damit an der Wunde wird hierdurch vermieden. Ein Abziehen des Trägermaterials von der Haut beeinträchtigt demzufolge die Hautwunde selbst nicht.

In weiterer Ausgestaltung der Erfindung weist die hydrophobe Struktur wenigstens eine hydrophobe Schicht auf, die an einer Unterseite des Trägermaterials oder an der Rückfläche der Wundauflage oder als separate Trennschicht zwischen Trägermaterial und Wundauflage vorgesehen ist. Als separate Trennschicht kann vorzugsweise ein mit Parafinöl getränktes textiles Flächengebilde, insbesondere in Form eines Gewebes, oder Fettgaze oder auch ein Depot eines hydrophoben, flüssigen oder fließfähigen Mediums vorgesehen sein, das beim Aufbringen des Wundpflasters auf die Wunde zwischen Wundauflage und Trägermaterial freigesetzt wird. Ein geeignetes Medium hierfür ist Vaseline. Vorteilhaft wird die hydrophobe Schicht oder Trennschicht nach der Applikation des Wundpflasters auf die Hautwunde gemeinsam mit der Entfernung des Trägermaterials entfernt, um die Atmungsaktivität der resorbierbaren Wundauflage nicht zu beeinträchtigen.

Erfindungsgemäß weist die Haltestruktur lösbare Verbindungsstellen zwischen Trägermaterial und Wundauflage auf, die die Wundauflage in nicht appliziertem Zustand am Trägermaterial sichern. Die lösbaren Verbindungsstellen dienen dazu, dass das Trägermaterial und die Wundauflage miteinander verbunden sind, solange das Wundpflaster nicht auf die Haut aufgeklebt ist. Die Verbindungsstellen der Haltestruktur können lediglich randseitig mit der Wundauflage zusammenwirken oder im Wesentlichen über eine gesamte Rückfläche der Wundauflage verteilt sein.

In weiterer Ausgestaltung der Erfindung sind die Verbindungsstellen Randbereichen der Wundauflage zugeordnet. Die Verbindungsstellen sind bei dieser Ausgestaltung ausschließlich an den Randbereichen der Wundauflage wirksam, wodurch sich im Wesentlichen linienförmige lösbare Verbindungen zwischen Wundauflage und Trägermaterial ergeben, die werkzeuglos manuell und mit geringem Kraftaufwand getrennt werden können.

In weiterer Ausgestaltung der Erfindung sind die Verbindungsstellen durch stoffschlüssige Verbindungsmittel, insbesondere durch Randabschnitte der Haftflächen des Trägermaterials, gebildet. Als stoffschlüssige Verbindungsmittel sind vorzugsweise Klebebereiche aus hautverträglichem Klebstoff vorgesehen. Besonders vorteilhaft ist es, die Wundauflage an ihren Randbereichen mit den Randabschnitten der bereits vorhandenen Haftflächen des Trägermaterials zur Fixierung des Wundpflasters auf der Haut zu verbinden. Die Haftflächen des Trägermaterials haben hierdurch eine Doppelfunktion, indem sie zum einen das Trägermaterial auf der Haut fixieren und zum anderen die lösbare Sicherung der Wundauflage an der Unterseite des Trägermaterials gewährleisten. Die Flächenabmessungen der Wundauflage sind für diese Ausgestaltung derart ausgeführt, dass die Wundauflage in ihrer Anlage an einer Unterseite des Trägermaterials die Randabschnitte der Haftflächen überdeckt. Damit ist die Wundauflage an wenigstens zwei gegenüberliegenden Randbereichen am Trägermaterial fixiert. Stoffschlüssige Verbindungsmittel sind insbesondere Klebeflächen oder thermisch aktivierte Fixierungsflächen wie insbesondere Schweißflächen.

In weiterer Ausgestaltung der Erfindung sind die Verbindungsstellen durch mechanisch wirksame Verbindungselemente, insbesondere durch Haftverschlusselemente, gebildet. Hierzu kann das Trägermaterial zumindest abschnittsweise im Bereich seiner Unterseite mit einer textilen Haken- oder Schlingenstruktur versehen sein. Die Wundauflage weist komplementär hierzu im Bereich ihrer der Wundauflagefläche gegenüberliegenden Rückfläche korrespondierende Haken- oder Schlingenstrukturelemente auf.

In weiterer Ausgestaltung der Erfindung ist zwischen Trägermaterial und Wundauflage ein medizinisch wirksames Depot vorgesehen, das bei einer Applikation des Wundpflasters aktivierbar ist. Das medizinisch wirksame Depot kann vorzugsweise in einer Folientasche vorgesehen sein, deren Außenhaut die hydrophobe Struktur zwischen Wundauflage und Trägermaterial bildet. Die Aktivierung des Depots bei einer Applikation des Wundpflasters bedeutet, dass die Aktivierung gemeinsam mit oder unmittelbar vor Aufbringung des Wundpflasters erfolgt. Vorzugsweise ist die als Depottasche dienende Folientasche hierzu auf ihrer der Wundauflage zugewandten Seite mit wenigstens einer mechanisch auftrennbaren Öffnung, vorzugsweise in Form einer Perforation, versehen. Das Depot wird durch wenigstens ein Medikament gebildet, das bei Aktivierung des Depots zur Wundauflage hin freigesetzt wird. Das Aufreissen der perforierten Öffnung erfolgt vorzugsweise durch Druckbeaufschlagung der als Verpackung dienenden Aufnahmetasche für das Depot.

In weiterer Ausgestaltung der Erfindung ist die Wundauflage mit eingelagerten medizinischen, eine Wundheilung fördernden Wirkstoffen versehen, die bei einer Applikation des Wundpflasters freisetzbar sind. Vorzugsweise sind antibakterielle Substanzen oder Arzneimittel in das Wundauflagematerial integriert. Die Integration kann über entsprechende Polymereigenschaften des Wundauflagematerials oder über Struktureigenschaften des Wundauflagematerials erfolgen. So können entsprechende Substanzen in eine Polymerstruktur des Wundauflagematerials eingebunden sein. Alternativ oder ergänzend kann eine textile Flächenstruktur des Wundauflagematerials so gestaltet sein, dass eine Tränkung der Wundauflage mit flüssigen Arzneimittel oder anderen medizinisch wirksamen Flüssigkeiten erfolgen kann.

In weiterer Ausgestaltung der Erfindung sind die Wundauflage und das Trägermaterial aus Materialien mit zueinander korrespondierenden Dehnungsvermögen gestaltet. Die Wundauflage weist vorzugsweise bei menschlicher Körpertemperatur eine besonders hohe Flexibilität und ein besonders hohes Dehnungsvermögen auf. Eine entsprechende Deformation kann plastisch und/oder elastisch erfolgen. Das Trägermaterial ist in entsprechender Weise flexibel und plastisch und/oder elastisch dehnbar. Bei der resorbierbaren Wundauflage kann dies durch Polymereigenschaften oder durch Struktureigenschaften der Wundauflage erzielt werden. Beim Trägermaterial werden die beschriebenen Flexibilitäts- und Dehnungseigenschaften vorzugsweise durch entsprechende Gestaltung einer textilen Struktur des Trägermaterials erzielt. Als geeignetes Trägermaterial ist insbesondere eine Gewebekonstruktion vorgesehen. Das Trägermaterial kann aber auch in Form eines Gewirkes oder eines Vliesstoffes oder einer elastischen Folie in geeigneter Weise elastisch oder plastisch verformbar sein. Vorzugsweise enthält eine entsprechende textile Flächenstruktur des Trägermaterials Elasthan- oder Polyamidfasern. Die elastische Verformbarkeit des Trägermaterials kann auch durch geeignete Polymereigenschaften einer Kunststofffolie für das Trägermaterial erreicht werden. Das Trägermaterial kann einen ein- oder mehrschichtigen Aufbau aufweisen.

In weiterer Ausgestaltung der Erfindung ist die Wundauflage mit einer Solltrennstruktur versehen, die insbesondere über eine Teilfläche der Wundauflage oder über eine Gesamtfläche der Wundauflage erstreckt ist. Die Solltrennstruktur, die sich wenigstens über einen Teilbereich einer Gesamtfläche der Wundauflage erstreckt, ist derart gestaltet, dass im Applikationsfall ein einfaches manuelles Abtrennen von über die Wundfläche der Hautwunde hinausragenden Bereichen der Wundauflage erzielbar ist, ohne dass hierfür ein Werkzeug wie insbesondere eine Schere oder ähnliches benötigt wird. Vorzugsweise verbleibt die Wundauflage nach Applikation des Wundpflasters auf die Hautwunde an den mit der Wundfläche verklebten Bereichen der Wundauflage als Einheit verbunden, wohingegen darüber hinausragende Abschnitte der Wundauflage, die im Bereich von gesunder oder bereits geheilter Haut überlappen, mit geringem Kraftaufwand werkzeuglos von Hand ablösbar sind. Vorzugsweise ist die Solltrennstruktur derart gestaltet und auf die Haltestruktur zwischen Wundauflage und Trägermaterial abgestimmt, dass ein Abziehen des Trägermaterials von der Wundauflage zwangsläufig auch die nicht oder nicht mehr zur Heilung benötigten Abschnitte der Wundauflage mit abtrennt.

In Ausgestaltung der Erfindung ist die resorbierbare Wundauflage, als textiles Flächengebilde, als polymere Flächenstruktur oder als polymerer Formkörper gestaltet und mit einer Solltrennstruktur versehen. Die Wundauflage wird auf die Wunde aufgebracht, verklebt aufgrund der Wundflüssigkeit mit der Wunde und verbleibt dort bis zur völligen Abheilung. Die erfindungsgemäße, resorbierbare Wundauflage ist vorteilhaft für die Versorgung kleiner bis mittelgroßer Hautwunden vorgesehen. Die Wundauflage besteht aus körperverträglichen Stoffen, die künstlich erzeugt oder aus der Natur gewonnen werden. Vorzugsweise ist die resorbierbare Wundauflage aus einem Wundauflagematerial entsprechend der EP 1919528 B1 oder entsprechend der EP 1181941 A2 hergestellt. Das Wundauflagematerial kann ein- oder mehrschichtig als polymere oder textile Flächenstruktur, insbesondere als Vliesstoff, oder als offenporiger Formkörper, insbesondere als Schaumstoff mit einer Polymerstruktur entsprechend dem mit der EP 1919528 B1 beschriebenen Formkörper gestaltet sein. Durch die erfindungsgemäße Lösung wird keine Anpassung der Größe der Wundauflage auf die Größe der Hautwunde vor der Applikation der Wundauflage benötigt. Vielmehr wird die Wundauflage mit ihrer vollständigen, vorgehaltenen Größe auf die Hautwunde aufgelegt. An den Stellen, an denen die Wundauflage auf gesunder Haut oder bereits geheilter Haut aufliegt, können entsprechende Wundauflageränder aufgrund der Solltrennstruktur in einfacher Weise manuell abgetrennt werden, nachdem die Wundauflage auf die Hautwunde aufgebracht wurde und über entsprechende getrocknete Wundflüssigkeit und Wundschorf mit der Wunde verklebt ist. Die Heilung der Hautwunde wird hierdurch nicht beeinträchtigt, da aufgrund der Solltrennstruktur lediglich Abschnitte der Wundauflage abgetrennt werden, die sich auf gesunden oder bereits abgeheilten Hautbereichen befinden.

In Ausgestaltung der Erfindung ist die Solltrennstruktur über eine Teilfläche der Wundauflage oder über eine Gesamtfläche der Wundauflage erstreckt. Falls die Solltrennstruktur lediglich über eine Teilfläche der Wundauflage erstreckt ist, sind entsprechende Teilflächenbereiche vorzugsweise außerhalb eines Zentrums der Wundauflage vorgesehen. Eine derartige Ausgestaltung geht davon aus, dass die Wundauflage im Wesentlichen zentrisch auf die zu behandelnde Hautwunde aufgelegt wird. Der zentrische Bereich der Hautwunde sollte vorzugsweise bis zur vollständigen Abheilung mit der Wundauflage in Kontakt bleiben, so dass in diesem Bereich keine vorzeitige Abtrennung der Wundauflage erfolgen muss und demzufolge die Wundauflage in diesem Bereich auch keine Solltrennstruktur aufweisen muss.

In weiterer Ausgestaltung der Erfindung ist die Solltrennstruktur durch punkt- und/oder linienförmige Perforation gestaltet. Die punkt- und/oder linienförmige Perforation ist so gewählt, dass entweder über die Höhe der Wundauflage oder über die Flächenerstreckung der Wundauflage lediglich eine teilweise Trennung des Wundauflagematerials erfolgt, so dass die Wundauflage ohne entsprechende mechanische Belastung in der einheitlichen und einteiligen Strukturform verbleibt.

In weiterer Ausgestaltung der Erfindung ist die Solltrennstruktur mechanisch durch Stanzen oder thermisch durch eine Laserbearbeitung hergestellt. Je nach der Gestaltung sowie nach polymerer oder struktureller Eigenschaften der Wundauflage wird das geeignete Bearbeitungsverfahren zur Herstellung der Solltrennstruktur ausgewählt, wobei die Eigenschaften der Wundauflage nicht oder nur unwesentlich verändert werden.

Die der Erfindung zugrunde liegende Aufgabe wird auch durch ein Set für die Herstellung wenigstens eines Wundpflasters zur Behandlung von Hautwunden gelöst, das mit wenigstens einem Trägermaterialzuschnitt und mit wenigstens einem größenmäßig auf den wenigstens einen Tägermaterialzuschnitt abgestimmten Wundauflagezuschnitt aus resorbierbarem Material sowie mit einer Umverpackung zur Aufnahme des wenigstens einen Trägermaterialzuschnittes und des wenigstens einen Wundauflagezuschnittes versehen ist, wobei der wenigstens eine Trägermaterialzuschnitt und der wenigstens eine Wundauflagezuschnitt als separate Teile in der Umverpackung angeordnet sind, und wobei dem Trägermaterialzuschnitt oder dem Wundauflagezuschnitt eine Haltestruktur zur lösbaren Verbindung jeweils eines Trägermaterialzuschnittes mit einem Wundauflagezuschnitt zur Herstellung eines Wundpflasters zugeordnet sind. Dieses Set ermöglicht die Bereitstellung des Trägermaterials und der resorbierbaren Wundauflage als separate Komponenten, die vom Bediener, insbesondere einem Klinikpersonal, einem behandelnden Arzt oder einer beliebigen dritten Person für einen entsprechenden Applikationsfall unter Bildung eines Wundpflasters zusammengefügt werden können. Vorteilhaft kann das Set mehrere Wundauflagezuschnitte zur Behandlung unterschiedlicher medizinischer Indikationen sowie Trägermaterialzuschnitte unterschiedlicher Größe, unterschiedlicher Materialien und mit unterschiedlichem elastischem Dehnungsvermögen bereit stellen, die je nach Bedarf und Applikationszweck vom jeweiligen Anwender modular zusammengefügt werden können. Je nach Einsatzzweck kann ein derartiges Set wie auch die einzelnen Komponenten in dem Set steril oder nicht steril ausgeführt sein. Unterschiedliche Wundauflagezuschnitte können insbesondere für Schürf- oder Risswunden, für Verbrennungswunden oder für durch Allergien verursachte Hautwunden im Set bereitgestellt werden. Die Haltestruktur, die jeweils einen entsprechenden Wundauflagezuschnitt mit einem darauf abgestimmten Trägermaterialzuschnitt lösbar verbindet, ist vorzugsweise gemäß einer der zuvor beschriebenen Ausgestaltungen ausgeführt.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Figur 1: zeigt schematisch eine Rückansicht eines Oberkörpers eines Patienten, auf dessen Rückenbereich eine Hautwunde mittels einer Ausführungsform eines erfindungsgemäßen Wundpflasters versorgt wurde,
- Figur 2: vergrößert schematisch eine Schnittdarstellung entlang der Schnittlinie II-II in Figur 1 ,
- Figur 3: eine Schnittdarstellung des Wundpflasters ähnlich Figur 2,
- Figur 4: einen Ausschnitt einer weiteren Ausführungsform eines erfindungsgemäßen Wundpflasters mit mechanisch wirksamen Verbindungsstellen zwischen Trägermaterial und Wundauflage,
- Figur 5: schematisch eine Draufsicht auf eine mit einer Hautoberfläche zu verbindende Unterseite einer weiteren Ausführungsform eines erfindungsgemäßen Wundpflasters,
- Figur 6: in gleicher Darstellungsweise wie Figur 5 eine weitere Ausführungsform eines erfindungsgemäßen Wundpflasters,
- Figur 7: schematisch und vergrößert eine Schnittdarstellung einer resorbierbaren Wundauflage des Wundpflasters nach Figur 6 entlang der Schnittlinie VII-VII in Figur 6,
- Figur 8: eine Draufsicht auf eine Ausführungsform einer erfindungsgemäßen resorbierbaren Wundauflage mit über Teilflächen verteilter Solltrennstruktur und
- Figur 9: eine Ausführungsform eines erfindungsgemäßen Sets für die Herstellung eines Wundpflasters zur Behandlung von Hautwunden.

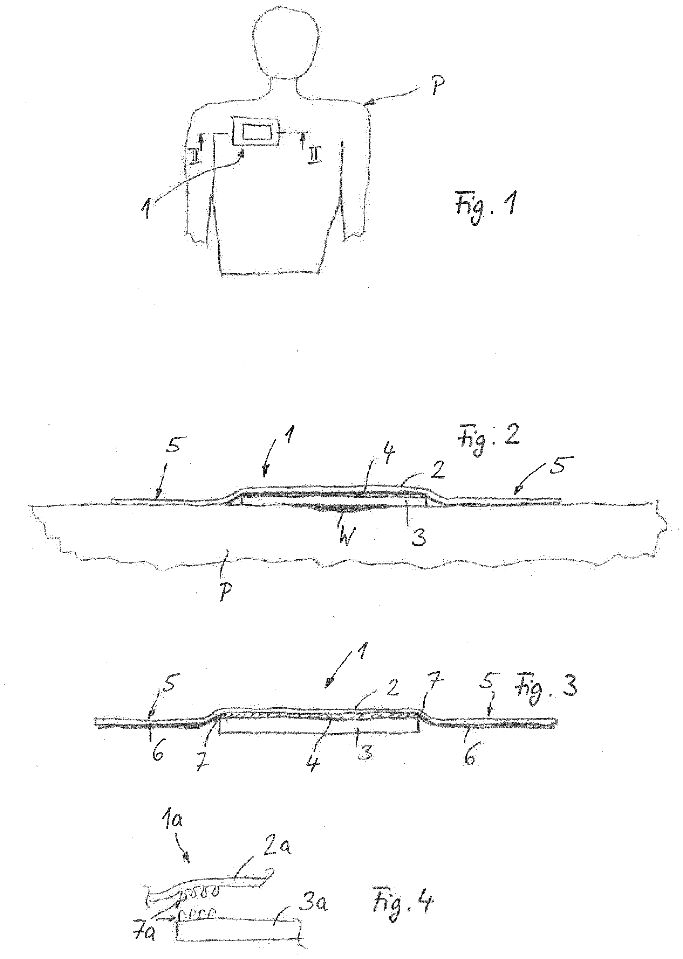

Ein menschlicher Patient P nach den Figuren 1 und 2 weist in einem Rückenbereich eine als Schnitt- oder Schürfwunde gestaltete Hautwunde W auf, die mittels eines Wundpflasters 1 versorgt ist. Das Wundpflaster 1 weist eine resorbierbare Wundauflage 3 auf, die bis zur Abheilung der Hautwunde W auf dieser verbleibt. Die Wundauflage 3 besteht aus resorbierbarem Material, das als Vliesstoff, als Gewebe, als Gewirke, als Folie, als Membran oder als Schaumstoff hergestellt sein kann und ein- oder mehrschichtig aufgebaut ist. Das Wundauflagematerial ist auf Polymerbasis (PGA, PLLA oder Copolymere aus PGA, PLLA oder andere Hydroxycarbonsäuren) aufgebaut. Das resorbierbare Wundauflagematerial hat eine antibakterielle Wirkung. Bei einer besonders bevorzugten Ausführungsform ist das Wundauflagematerial auf Suprathelpolymerbasis aufgebaut, das durch Degradation des Polymers unter Freisetzung von Monomeren wundheilungsfördernde Wirkung hat. Ergänzend kann das Wundauflagematerial zur Verstärkung der Wundheilungswirkung mit eingelagerten Arzneimitteln und/oder mit antibakteriellen Substanzen versehen sein, wobei vorzugsweise eine lokale Freisetzung von Wirkstoffen erfolgt. Die resorbierbare Wundauflage ist aus körperverträglichen Stoffen aufgebaut, die im menschlichen Körper vollständig durch Stoffwechsel abgebaut werden können. Das Wundauflagematerial weist eine Wasserdampfdurchlässigkeit auf, die wenigstens einer Wasserdampfdurchlässigkeit der Haut entspricht.

Die Wundauflage 3 wird mit ihrer Wundfläche auf die Hautwunde W aufgebracht. Im Bereich ihrer Rückfläche ist die Wundauflage 3 einer Unterseite eines flächigen und flexiblen Trägermaterials 2 zugeordnet, dessen Fläche eine Grundfläche der Wundauflage 3 auf allen Seiten überragt. Die Wundauflage 3 ist wenigstens an zwei gegenüberliegenden Randbereichen ihrer Rückfläche mit der Unterseite des Trägermaterials 2 im Bereich von Verbindungsstellen 7 lösbar verbunden. Die Verbindungsstellen 7 werden durch Haftflächenabschnitte von Haftflächen 6 im Bereich der Unterseite des Trägermaterials 2 gebildet, wobei die Haftflächen 6 an Seitenabschnitten 5 des Trägermaterials 2 vorgesehen sind, die seitlich neben der Wundauflage 3 angeordnet sind. Als Haftflächen 6 dienen Klebeflächen einer Haftklebeschicht, die aus hautverträglichem Klebstoff hergestellt ist und mit der Unterseite des Trägermaterials 2 im Bereich der Seitenabschnitte 5 verbunden ist.

Die untere Seite des Trägermaterials 2 ist in dem Flächenbereich, der unmittelbar oberhalb der Rückfläche der Wundauflage 3 vorgesehen ist, mit einer hydrophoben Schicht 4 versehen, die beim dargestellten Ausführungsbeispiel mit der Unterseite des Trägermaterials zweiflächig verbunden ist. Durch die hydrophobe Schicht 4 weist das Trägermaterial 2 in diesem Flächenbereich kein Aufsaugvermögen für Wundflüssigkeit auf.

Bei alternativen Ausführungsbeispielen wird die hydrophobe Schicht durch eine sowohl vom Trägermaterial 2 als auch von der Wundauflage 3 getrennte Zwischenschicht, insbesondere in Form von Fettgaze, gebildet. Als hydrophobe Schicht kann auch eine Vaseline-Schicht oder eine ähnliche Fett- oder Ölschicht vorgesehen sein, die in einem Depot zwischen Trägermaterial 2 und Wundauflage 3 angeordnet ist und erst durch Druckbelastung bei Applikation des Wundpflasters 1 freigesetzt wird und sich zwischen dem Trägermateriat 2 und der Wundauflage 3 verteilt.

In allen Fällen dient die hydrophobe Struktur oder die hydrophobe Schicht dazu, das Verkleben des Trägermaterials mit der Wundauflage aufgrund der Aufnahme von Wundflüssigkeit im Bereich des Trägermaterials zu vermeiden.

Die Wundauflage 3 wird über die Klebeflächenabschnitte im Bereich der Verbindungsstellen 7 an ihren Randbereichen im Bereich der Unterseite des Trägermaterials 2 gehalten. Diese Klebeflächenabschnitte sind so schmal gestaltet, dass zwar eine ausreichende Fixierung der resorbierbaren Wundauflage 3 an dem Trägermaterial 2 des Wundpflasters 1 in unbenutztem, nicht appliziertem Zustand gegeben ist. Sobald aber das Wundpflaster 1 gemäß in Figuren 1 und 2 auf eine entsprechende Hautwunde W aufgebracht ist und das Trägermaterial 2 durch Abziehen im Bereich der Seitenabschnitte 5 und der Haftflächen 6 nach ein bis zwei Tagen entfernt wird, löst sich das Trägermaterial 5 im Bereich der Verbindungsstellen 7 von der auf der Hautwunde W verbleibenden Wundauflage, ohne diese zu beschädigen. Es erfolgt daher keine Irritation der Wundheilung durch Abziehen des Trägermaterials 2. Das Abziehen des Trägermaterials 2 bewirkt eine erhöhte Atmungsfähigkeit der auf der Hautwunde W verbliebenen Wundauflage 3, wodurch die Heilungswirkung beschleunigt wird.

Die Wundauflage 3 weist wenigstens bei Körpertemperatur ein hohes plastisches oder elastisches Dehnungsvermögen auf. Auch das Trägermaterial 2 ist elastisch verformbar. Das Trägermaterial 2 ist vorzugsweise durch eine textile Flächenstruktur in Form eines Gewebes, eines Gewirkes oder eines Vliesstoffes hergestellt und ist insbesondere durch Elasthan- oder Polyamidfilamente elastisch verformbar. Bei einem nicht dargestellten Ausführungsbeispiel besteht das Trägermaterial 2 aus einer elastischen Kunststofffolie, insbesondere aus PVC oder PUR.

Beim Ausführungsbeispiel gemäß den Figuren 1 bis 3 verbleibt die Wundauflage 3 nach dem Abziehen des Trägermaterials 2 einschließlich Haftflächen 6 und hydrophober Schicht 4 in im Wesentlichen unveränderter Flächenform auf der Hautwunde W, bis die Wunde verheilt ist. Wundauflagenabschnitte, die zum Zeitpunkt der vollständigen Heilung noch nicht durch Stoffwechselaktivität des menschlichen Körpers abgebaut sind, können in einfacher Weise, gegebenenfalls gemeinsam mit Wundschorf, entfernt werden. Um die Entfernbarkeit zu verbessern, kann eine Anfeuchtung insbesondere mit Wasser oder Öl erfolgen.

Bei der Ausführungsform eines Wundpflasters 1a gemäß Figur 4 werden die Verbindungsstellen 7a an den Randbereichen der Rückfläche der resorbierbaren Wundauflage 3a durch mechanisch wirksame, lösbare Verbindungselemente, vorliegend in Form von Haftverschlusselementen, gebildet. Dabei ist im Bereich einer Unterseite des Trägermaterials 2a eine Schlingenbildung aus der Flächenstruktur des Trägermaterials 2a heraus vorgesehen. Die Rückfläche der Wundauflage 3a weist in ihren Randbereichen korrespondierende Hakenelemente auf, die in die Struktur der Wundauflage 3a eingelagert sind und durch Zusammenfügen von Trägermaterial 2a und Wundauflage 3a mit den Schlingen des Trägermaterials 2 in Wirkverbindung gelangen.

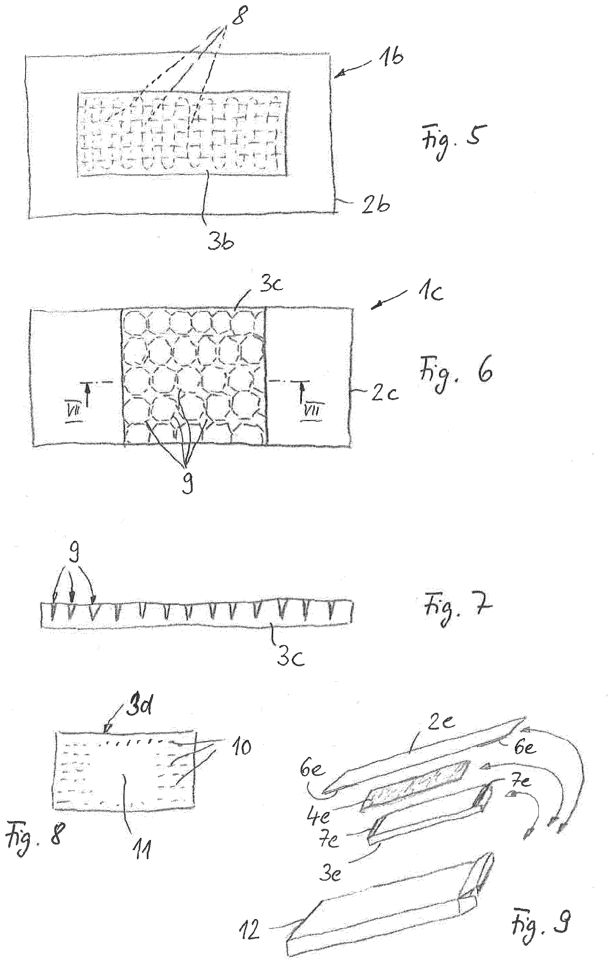

Bei der Ausführungsform nach Figur 5 weist ein Wundpflaster 1b eine im Bereich einer Unterseite eines Trägermaterials 2b lösbar angebrachte, resorbierbare Wundauflage 3b auf, die über ihre gesamte Grundfläche mit einer durch linienförmige Perforation gebildeten, karoartigen Solltrennstruktur 8 versehen ist. Auch bei der Ausführungsform nach Figur 5 überragt das Trägermaterial 2b die Grundfläche der Wundauflage 3b allseitig.

Bei dem Wundpflaster 1c nach Figur 6 weist das Trägermaterial 2c die gleiche Breite auf (in Zeichnungshochrichtung gesehen) - wie die Wundauflage 3c. Das Trägermaterial 2c ist lediglich bezüglich seiner Länge größer gestaltet als die Wundauflage 3c, so dass das Trägermaterial 2c die Wundauflage 3c zu zwei gegenüberliegenden Seiten hin überragt. Auch bei dem Wundpflaster 1c nach Figur 6 ist die resorbierbare Wundauflage 3c lösbar mit einer Unterseite des Trägermaterials 2c versehen, um analog zu der anhand der Figuren 2 und 3 beschriebenen Ausführungsform einen Verbleib der Wundauflage 3c auf einer Hautwunde zu ermöglichen, auch wenn das Trägermaterial 2c abgezogen wird. Das Wundpflaster 1c stellt einen Zuschnitt eines langen, zu einer Rolle aufwickelbaren Wundpflasterbandes dar, bei dem das Wundauflagematerial korrespondierend bandförmig sich über die gesamte Länge des bandförmigen Trägermaterials erstreckt und mit diesem verbunden ist. Sowohl bei dem Wundpflaster 1b nach Figur 5 als auch bei dem Wundpflaster 1c nach Figur 6 entsprechen Aufbau und Funktion im übrigen der zuvor anhand der Figuren 2 und 3 beschriebenen Ausführungsform. Zwischen der Wundauflage 3b, 3c und dem zugewandten Flächenbereich der Unterseite des Trägermaterials 2b, 2c ist eine hydrophobe Struktur analog der zuvor beschriebenen Ausführungsformen vorgesehen. Bei dem Wundpflaster 1c nach Figur 6 ist die Wundauflage 3c mit einer Solltrennstruktur 9 versehen, die durch Perforationen in sechseckiger Rhombenform, wie anhand der Figur 6 erkennbar, gebildet ist. Anhand der Figur 7 ist erkennbar, dass die Solltrennstruktur 9 sich lediglich über einen Teil der Dicke der Wundauflage 3c erstreckt, so dass die Wundauflage in nicht appliziertem Zustand in ihrer einheitlichen Flächenform an der Unterseite des Trägermaterials 2c fixiert bleibt. Die Solltrennstrukturen 8 und 9 bilden Sollriss- oder Sollbruchstellen, je nach Materialgestaltung der Wundauflage 3b, 3c. Diese Solltrennstruktur hat den Vorteil, dass die resorbierbare Wundauflage an den Stellen, an denen keine Verklebung mit der Hautwunde besteht, in einfacher Weise manuell und ohne die Zuhilfenahme von Werkzeugen entfernt werden kann, wobei die Kräfte zum Trennen der Wundauflageabschnitte im Bereich der Sollriss- oder Sollbruchstellen so gering sind, dass keine Dehnungsbelastung der Wundauflage und demzufolge keine Irritation der Wundheilung erfolgt. Die mit der Wunde verklebten Abschnitte der Wundauflage verbleiben auf der Wunde, bis die Wundheilung zum Abschluss gekommen ist.

Figur 8 zeigt eine resorbierbare Wundauflage 3d, die nicht Teil eines Wundpflasters ist, sondern vielmehr ohne ein zugehöriges Trägermaterial auf eine entsprechende Hautwunde aufgelegt wird. Die Wundauflage 3d ist lediglich in Teilbereichen ihrer Grundfläche mit einer Solltrennstruktur 10 versehen. Ein zentraler, mittig angeordneter Abschnitt 11 der Wundauflage 3d weist keine Solltrennstruktur auf, sondern besitzt vielmehr eine einheitliche Materialdicke.

Eine weitere resorbierbare Wundauflage, die aus einem der zuvor aufgeführten Wundauflagematerialien hergestellt sein kann, wird ebenfalls zur direkten Wundbehandlung analog der Ausführungsform nach Figur 8 vorgesehen und weist eine über ihre gesamte Grundfläche durchgängige Solltrennstruktur analog der Ausführungsform nach den Figuren 5 bis 7 auf. Die beschriebenen Solltrennstrukturen der Wundauflagen ermöglichen das einfache, manuelle Ablösen von den Teilen der Wundauflage, die entweder wunde Hautbereiche oder bereits abgeheilte Hautbereiche überdecken, ohne dass durch dieses Abtrennen dieser Teilbereiche der mit der Wunde verklebte Teilbereich der Wundauflage belastet wird. Die Wundheilung wird hierdurch nicht beeinträchtigt.

Anhand der Figur 9 ist ein Set zur Lagerung von Komponenten zur Herstellung eines Wundpflasters vorgesehen. Dabei sind drei Komponenten vorgesehen, die voneinander getrennt bereitgestellt sind, die aber in einfacher Weise zu einem Wundpflaster manuell zusammengefügt werden können. Die eine Komponente wird durch ein flexibles, flächiges Trägermaterial 2e gebildet, das an zwei gegenüberliegenden Seitenabschnitten unterseitig mit jeweils einer Haftfläche 6e versehen ist. Die zweite Komponente wird durch eine hydrophobe Struktur 4e, vorliegend in Form einer Fettgaze, gebildet, und die dritte Komponente ist eine Wundauflage 3e, die an gegenüberliegenden Randbereichen ihrer Rückfläche mit jeweils einer streifenförmigen Verbindungsstelle 7e versehen ist. Sowohl die Wundauflage 3e als auch das Trägermaterial 2e und die hydrophobe Struktur 4e sind jeweils als Zuschnitte bereitgestellt, die in ihren Größen aufeinander abgestimmt sind. Alle Komponenten weisen - auf ihre Grundfläche bezogen - die gleiche Breite auf. Die Länge der Fettgaze 4e entspricht dem Abstand der streifenförmigen Verbindungsstellen 7e im Bereich der Rückfläche der Wundauflage 3e zueinander. Der Abstand der gegenüberliegenden Haftflächen 6e des Trägermaterialzuschnittes ist etwas geringer als die Länge der Wundauflage 3e, so dass die Wundauflage 3e zur Herstellung eines Wundpflasters unter Zwischenlage der hydrophoben Struktur 4e an die Unterseite des Trägermaterialzuschnittes angefügt werden kann, wodurch die Wundauflage 3e im Bereich der streifenförmigen Verbindungsstellen 7e lösbar mit der Unterseite des Trägermaterialzuschnittes verbunden ist. Jede der Komponenten kann in nicht näher dargestellter Weise mit einer folienförmigen Umhüllung versehen sein. Alle drei Komponenten sind untergebracht in einer vorliegend schachtelförmigen Umverpackung 12.

## Patentansprüche

1. Wundpflaster mit einer Wundauflage, die im Applikationsfall mittels einer Wirkfläche mit einer Hautwunde in Kontakt gelangt, sowie mit einem flächigen Trägermaterial, das die Wundauflage auf einer zur Wirkfläche der Wundauflage entgegengerichteten Rückfläche zumindest abschnittsweise überdeckt, und das die Wundauflage mit wenigstens zwei zumindest weitgehend gegenüberliegenden Haftflächen überragt, die zur Fixierung des Trägermaterials auf menschlicher oder tierischer Haut vorgesehen sind, **dadurch gekennzeichnet, dass** die Wundauflage (3 bis 3e) aus resorbierbarem Material besteht und mittels einer Haltestruktur (7 bis 7e) lösbar mit dem Trägermaterial (2 bis 2) verbunden ist und die Haltestruktur lösbare Verbindungsstellen (7 bis 7e) zwischen Trägermaterial (2 bis 2e) und Wundauflage (3 bis 3e) aufweist, die die Wundauflage in nicht appliziertem Zustand am Trägermaterial sichern.

2. Wundpflaster nach Anspruch 1, **dadurch gekennzeichnet, dass** wenigstens in einem Überdeckungsbereich zwischen dem Trägermaterial (2 bis 2e) und der Wundauflage (3 bis 3e) eine hydrophobe Struktur (4, 4e) vorgesehen ist, die im Applikationsfall eine Aufnahme von Wundflüssigkeit durch das Trägermaterial (2 bis 2e) verhindert.

3. Wundpflaster nach Anspruch 2, **dadurch gekennzeichnet, dass** die hydrophobe Struktur wenigstens eine hydrophobe Schicht (4) aufweist, die an einer Unterseite des Trägermaterials (2) oder an der Rückfläche der Wundauflage oder als separate Trennschicht (4e) zwischen Trägermaterial und Wundauflage vorgesehen ist.

4. Wundpflaster nach Anspruch 1, **dadurch gekennzeichnet, dass** die Verbindungsstellen (7, 7a, 7e) Randbereichen der Wundauflage (3, 3a, 3e) zugeordnet sind.

5. Wundpflaster nach Anspruch 4, **dadurch gekennzeichnet, dass** die Verbindungsstellen durch stoffschlüssige Verbindungsmittel, insbesondere durch Randabschnitte der Haftflächen (6) des Trägermaterials (2), gebildet sind.

6. Wundpflaster nach Anspruch 1, **dadurch gekennzeichnet, dass** die Verbindungsstellen (7a) durch mechanisch wirksame Verbindungselemente, insbesondere durch Haftverschlusselemente, gebildet sind.

7. Wundpflaster nach Anspruch 1, **dadurch gekennzeichnet, dass** ein medizinisch wirksames Depot zwischen Trägermaterial und Wundauflage vorgesehen ist, das bei einer Applikation des Wundpflasters aktivierbar ist.

8. Wundpflaster nach Anspruch 1, **dadurch gekennzeichnet, dass** die Wundauflage mit eingelagerten medizinischen, eine Wundheilung fördernden Wirkstoffen versehen ist, die bei einer Applikation des Wundpflasters freisetzbar sind.

9. Wundpflaster nach Anspruch 1, **dadurch gekennzeichnet, dass** die Wundauflage (3 bis 3e) und das Trägermaterial (2 bis 2e) aus Materialien mit zueinander korrespondierendem Dehnungsvermögen gestaltet sind.

10. Wundpflaster nach einem der vorhergehenden Ansprüche, **dadurch gekennzeichnet, dass** die Wundauflage (3b, 3c, 3d) mit einer Solltrennstruktur (8 bis 10) versehen ist, die insbesondere über eine Teilfläche der Wundauflage oder über eine Gesamtfläche der Wundauflage erstreckt ist.

11. Wundpflaster nach Anspruch 10, wobei die resorbierbare Wundauflage als textiles Flächengebilde oder als polymere Flächenstruktur oder als polymerer Formkörper gestaltet ist.

12. Wundpflaster nach Anspruch 11, **dadurch gekennzeichnet, dass** die Solltrennstruktur (8 bis 10) über eine Teilfläche der Wundauflage (3b bis 3d) oder über eine Gesamtfläche der Wundauflage erstreckt ist.

13. Wundpflaster nach Anspruch 12, **dadurch gekennzeichnet, dass** die Solltrennstruktur (8 bis 10) durch punkt- und/oder linienförmige Perforation gestaltet ist.

14. Set für die Herstellung wenigstens eines Wundpflasters zur Behandlung von Hautwunden gemäß einem der Ansprüche 1 bis 13, mit wenigstens einem Trägermaterialzuschnitt (2e) und wenigstens einem größenmäßig auf den wenigstens einen Trägermaterialzuschnitt (2e) abgestimmten Wundauflagezuschnitt (3e) aus resorbierbarem Material, sowie mit einer Umverpackung zur Aufnahme des wenigstens einen Trägermaterialszuschnittes sowie des wenigstens einen Wundauflagezuschnittes, wobei der wenigstens eine Trägermaterialzuschnitt und der wenigstens eine Wundauflagezuschnitt als separate Teile in der Umverpackung (12) angeordnet sind, und wobei dem Trägermaterialzuschnitt (2e) oder dem Wundauflagezuschnitt (3e) eine Haltestruktur (7e) zur lösbaren Verbindung jeweils eines Wundauflagezuschnittes mit einem Trägermaterialzuschnitt zur Herstellung des Wundpflasters zugeordnet sind und die Haltestruktur lösbare Verbindungsstellen (7 bis 7e) zwischen Trägermaterial (2 bis 2e) und Wundauflage (3 bis 3e) aufweist, die die Wundauflage in nicht appliziertem Zustand am Trägermaterial sichern.

## Claims

1. An adhesive bandage comprising a wound dressing which, in the case of application, comes into contact with a skin wound by means of an active surface, and comprising a flat carrier material which covers the wound dressing at least in sections on a rear surface facing away from the active surface of the wound dressing and which projects beyond the wound dressing by at least two at least largely opposite adhesive surfaces which are provided for fixing the carrier material on human or animal skin, **characterized in that** the wound dressing (3 to 3e) is made from a resorbable material and is detachably connected to the carrier material (2 to 2) by means of a holding structure (7 to 7e), and the holding structure includes detachable connection points (7 to 7e) between the carrier material (2 to 2e) and the wound dressing (3 to 3e) which retain the wound dressing in the non-applied state to the carrier material.

2. The adhesive bandage according to claim 1, **characterized in that** at least in a covering region between the carrier material (2 to 2e) and the wound dressing (3 to 3e), a hydrophobic structure (4, 4e) is provided which prevents absorption of wound exudate by the carrier material (2 to 2e) in the case of application.

3. The adhesive bandage according to claim 2, **characterized in that** the hydrophobic structure includes at least one hydrophobic layer (4) which is provided on a lower side of the carrier material (2) or on the rear surface of the wound dressing or as a separate dividing layer (4e) between the carrier material and the wound dressing.

4. The adhesive bandage according to claim 1, **characterized in that** the connection points (7, 7a, 7e) are associated with edge regions of the wound dressing (3, 3a, 3e).

5. The adhesive bandage according to claim 4, **characterized in that** the connection points are formed by means producing substance-to-substance joints, in particular by edge portions of the adhesive surfaces (6) of the carrier material (2).

6. The adhesive bandage according to claim 1, **characterized in that** the connection points (7a) are formed by mechanically effective connecting elements, in particular by hook-and-loop fastener elements.

7. The adhesive bandage according to claim 1, **characterized in that** a medically active depot is provided between the carrier material and the wound dressing and is activatable upon an application of the adhesive bandage.

8. The adhesive bandage according to claim 1, **characterized in that** the wound dressing is provided with embedded medical active ingredients which promote wound healing and are releasable upon an application of the adhesive bandage.

9. The adhesive bandage according to claim 1, **characterized in that** the wound dressing (3 to 3e) and the carrier material (2 to 2e) are formed from materials having expansibilities corresponding to each other.

10. The adhesive bandage according to any of the preceding claims, **characterized in that** the wound dressing (3b, 3c, 3d) is provided with a predetermined separating structure (8 to 10) which extends in particular over a partial area of the wound dressing or over a total area of the wound dressing.

11. The adhesive bandage according to claim 10, wherein the resorbable wound dressing is configured as a textile web or as a polymeric flat structure or as a polymeric shaped body.

12. The adhesive bandage according to claim 11, **characterized in that** the predetermined separating structure (8 to 10) extends over a partial area of the wound dressing (3b to 3d) or over a total area of the wound dressing.

13. The adhesive bandage according to claim 12, **characterized in that** the predetermined separating structure (8 to 10) is formed by point-shaped and/or linear perforation.

14. A set for manufacturing at least one adhesive bandage for the treatment of skin wounds according to any of claims 1 to 13, comprising at least one carrier material blank (2e) and at least one wound dressing blank (3e) made from a resorbable material and adapted in terms of size to the at least one carrier material blank (2e), and comprising an outer packaging for receiving the at least one carrier material blank and the at least one wound dressing blank, the at least one carrier material blank and the at least one wound dressing blank being arranged as separate parts in the outer packaging (12), and the carrier material blank (2e) or the wound dressing blank (3e) having a holding structure (7e) associated with it for detachably connecting one respective wound dressing blank to one carrier material blank for manufacturing the adhesive bandage, and the holding structure having detachable connection points (7 to 7e) between the carrier material (2 to 2e) and the wound dressing (3 to 3e) which retain the wound dressing in the non-applied state to the carrier material.

## Revendications

1. Pansement pour plaie, présentant un revêtement pour plaie qui, dans le cas d'une application, parvient en contact avec une plaie cutanée par l'intermédiaire d'une surface active, et présentant une matière support plane qui recouvre le revêtement pour plaie au moins par tronçons sur une face arrière détournée de la surface active du revêtement pour plaie, et qui dépasse le revêtement pour plaie avec au moins deux surfaces adhésives qui sont au moins en grande partie opposées et qui sont prévues pour la fixation de la matière support sur la peau humaine ou animale, **caractérisé en ce que** le revêtement pour plaie (3 à 3e) est réalisé en une matière apte à être résorbée et est relié de manière détachable à la matière support (2 à 2) au moyen d'une structure de retenue (7 à 7e), et **en ce que** la structure de retenue présente des points de liaison (7 à 7e) détachables entre la matière support (2 à 2e) et le revêtement pour plaie (3 à 3e), lesquels fixent le revêtement pour plaie sur la matière support à l'état non appliqué.

2. Pansement pour plaie selon la revendication 1, **caractérisé en ce qu'**une structure hydrophobe (4, 4e) empêchant une absorption de liquide de la plaie par la matière support (2 à 2e) est prévue au moins dans une zone de recouvrement entre la matière support (2 à 2e) et le revêtement pour plaie (3 à 3e).

3. Pansement pour plaie selon la revendication 2, **caractérisé en ce que** la structure hydrophobe présente au moins une couche hydrophobe (4) qui est prévue d'un côté inférieur de la matière support (2) ou sur face arrière du revêtement pour plaie ou sous forme de couche de séparation (4e) distincte entre la matière support et le revêtement pour plaie.

4. Pansement pour plaie selon la revendication 1, **caractérisé en ce que** les points de liaison (7, 7a, 7e) sont associés à des zones de bord du revêtement pour plaie (3, 3a, 3e).

5. Pansement pour plaie selon la revendication 4, **caractérisé en ce que** les points de liaison sont formés par des moyens de liaison par coopération de matières, en particulier par des tronçons de bord des surfaces adhésives (6) de la matière support (2).

6. Pansement pour plaie selon la revendication 1, **caractérisé en ce que** les points de liaison (7a) sont formés par des éléments de liaison à effet mécanique, en particulier par des éléments de fermeture adhésifs.

7. Pansement pour plaie selon la revendication 1, **caractérisé en ce qu'**un dépôt à effet médical qui est apte à être activé lors d'une application du pansement pour plaie est prévu entre la matière support et le revêtement pour plaie.

8. Pansement pour plaie selon la revendication 1, **caractérisé en ce que** le revêtement pour plaie est pourvu de substances actives médicales incorporées qui favorisent une cicatrisation de la plaie et qui sont aptes à être libérées lors d'une application du revêtement pour plaie.

9. Pansement pour plaie selon la revendication 1, **caractérisé en ce que** le revêtement pour plaie (3 à 3e) et la matière support (2 à 2e) sont réalisés en matières présentant des expansibilités qui correspondent l'une à l'autre.

10. Pansement pour plaie selon l'une des revendications précédentes, **caractérisé en ce que** le revêtement pour plaie (3b, 3c, 3d) est pourvu d'une structure destinée à la séparation (8 à 10) qui s'étend en particulier sur une surface partielle du revêtement pour plaie ou sur une surface totale du revêtement pour plaie.

11. Pansement pour plaie selon la revendication 10, le revêtement pour plaie apte à être résorbé étant réalisé sous forme de formation plane textile ou de structure plane polymère ou de corps formé polymère.

12. Pansement pour plaie selon la revendication 11, **caractérisé en ce que** la structure destinée à la séparation (8 à 10) s'étend sur une surface partielle du revêtement pour plaie (3b à 3d) ou sur une surface totale du revêtement pour plaie.

13. Pansement pour plaie selon la revendication 12, **caractérisé en ce que** la structure destinée à la séparation (8 à 10) est réalisée par une perforation en forme de point et/ou de ligne.

14. Ensemble pour la fabrication d'au moins un pansement pour plaie pour le traitement de plaies cutanées selon l'une des revendications 1 à 13, comprenant au moins une découpe de matière support (2e) et au moins une découpe de revêtement pour plaie (3e) en matière apte à être résorbée, dont la taille est adaptée à ladite au moins une découpe de matière support (2e), et comprenant une enveloppe pour le logement de ladite au moins une découpe de matière support et de ladite au moins une découpe de revêtement pour plaie, ladite au moins une découpe de matière support et ladite au moins une découpe de revêtement pour plaie étant agencées dans l'enveloppe (12) sous forme de pièces séparées, et une structure de retenue (7e) pour la liaison détachable d'une découpe de revêtement pour plaie respective à une découpe de matière support pour la fabrication du pansement pour plaie étant associée à la découpe de matière support (2e) ou à la découpe de revêtement pour plaie (3e), et la structure de retenue présentant des points de liaison détachables (7 à 7e) entre la matière support (2 à 2e) et le revêtement pour plaie (3 à 3e), lesquels fixent le revêtement pour plaie sur la matière support à l'état non appliqué.
